# EUROPEAN PATENT SPECIFICATION

(11) **EP 3 395 277 B1**
(45) Date of publication and mention of the grant of the patent: **13.03.2024**
(21) Application number: 18169440.7
(22) Date of filing: 26.04.2018
(51) Int. Cl.: A61B 18/14, A61B 90/00, A61B 17/00, A61B 18/00, A61B 34/20, A61B 18/12, A61B 34/10

(54) **SYSTEMS FOR MAP-GUIDED AUTOMATIC CARDIAC ABLATION**
SYSTEME ZUR KARTENGEFÜHRTEN AUTOMATISCHEN HERZABLATION
SYSTÈMES D'ABLATION CARDIAQUE AUTOMATIQUE GUIDÉE PAR PLANS

(30) Priority: 27.04.2017 US 201715499484
(43) Date of publication of application: 31.10.2018
(73) Proprietor: Biosense Webster (Israel) Ltd., Yokneam 2066717 (IL)
(72) Inventor: GOVARI, Assaf, 2066717 Yokneam (IL); ALTMANN, Andres Claudio, 2066717 Yokneam (IL)
(74) Representative: Carpmaels & Ransford LLP

(56) References cited:
- EP-A1- 2 591 722
- US-A1- 2007 083 193
- US-A1- 2008 300 588
- US-A1- 2010 312 094
- US-A1- 2015 065 899

## Description

### BACKGROUND

Patent Application Publication US2015/0065899A1 discloses transducer-based systems and methods configured to display a graphical representation of a transducer-based device, the graphical representation including graphical elements corresponding to transducers of the transducer-based device, and also including between graphical elements respectively associated with a set of the transducers and respectively associated with a region of space between the transducers of the transducer-based device. Selection of graphical elements and/or between graphical elements can cause activation of the set of transducers associated with the selected elements.

### SUMMARY

The invention is defined by claims 1 and 8. Further embodiments of the invention are defined by the dependent claims.

### BRIEF DESCRIPTION OF THE DRAWINGS

The following detailed description of the embodiments of the present invention will be better understood when read in conjunction with the appended drawings. For the purpose of illustration, there are shown in the drawings embodiments which are presently preferred. It is understood, however, that the invention is not limited to the precise arrangements and instrumentalities shown. In the drawings:
FIG. 1 is a schematic representation of a system for performing automatic cardiac ablation.
FIG. 2 illustrates a screen display of an electro-anatomic map and a lasso catheter.
FIGS. 3A - 3B are views of the heart marked with target lines (FIG. 3A) and target sites (FIG. 3B) for automatic cardiac ablation
FIGS. 4A - 4B are views of the left atrium of the heart. FIG. 4A is a view a basket catheter inserted in the left atrium for performing automatic cardiac ablation. FIG. 4B is a view of an ablation line in the heart following the ablation procedure.
FIG. 5 is a flow chart illustrating a method for the map-guided cardiac ablation performed automatically, which method alone is not within the scope of the appended claims.

### DETAILED DESCRIPTION OF THE PREFERRED EMBODIMENTS

The present disclosure relates to cardiac ablation, and specifically, to systems and processes for map-guided cardiac ablation performed automatically.

Cardiac disorders, such as arrhythmias, are associated with abnormal conductivity of heart tissue. Procedures for treating the cardiac disorders include identifying the origin of the signals causing the arrhythmia and disrupting the conductivity pathway for the erroneous signals. It is possible to interrupt the propagation of the undesirable electric signals by selectively ablating cardiac tissue. Cardiac ablation is typically performed by applying alternating currents to electrodes for heating and destroying the target tissue to form non-conducting lesions. Ablation systems are known in the art. Physicians use ablation systems to track physiological and electrophysiological parameters, such as atrial chamber size, myocardial wall thickness, tissue electrical impedance and atrial contraction, and modulate ablation energy pulses by adaptively adjusting ablation parameters during electrical treatment including electrical pulse length and ablation time.

However, known ablation systems are based on knowledge and subjective assessments made by medical personal, and are prone to errors. A better system for identifying and ablating target sites is needed.

A system is provided that enables the physicians to manually or automatically select ablation sites by using a graphic-user interface (GUI) map of the heart, wherein the system automatically performs the ablation procedure. In one aspect, the system enables the physicians to pre-define the treatment according to the specific parameters, such as tissue thickness, presence of scar tissue, and safety concerns.

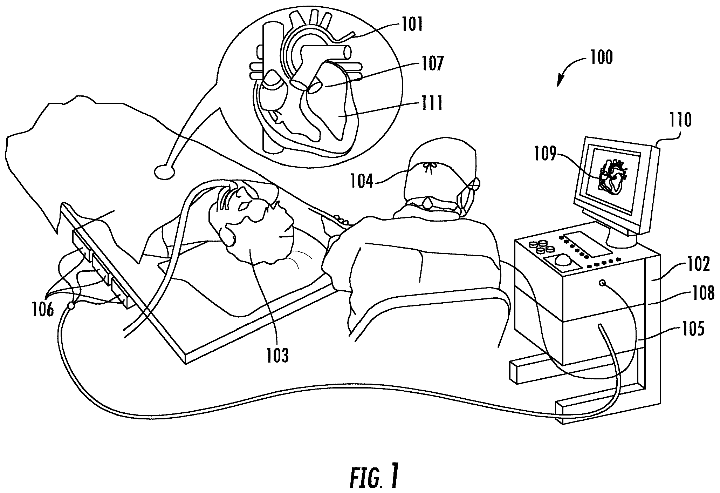

FIG. 1 is a schematic illustration of an exemplary system 100 for performing automatic cardiac ablation in a heart of a patient 103 according to one or more embodiments of the present invention. System 100 comprises a control console 102 that includes a processing device 108. Processing device 108 may include a processor 105 and a display device which may include a display 110 (such as a monitor and/or screen). Processing device 108 may include one or more processors each configured to process the ECG signals from the catheter. Each processor 105 of processing device 108 may be configured to record ECG signals over time, filter ECG signals, map ECG signals, combine ECG signal information, map and interpolate mapping information, etc. Processor 105 may comprise appropriate signal processing circuits for receiving signals from a catheter 101. Catheter 101 may be coupled to processing device 108, which enables the physician 104 to observe and regulate the functions of the catheter 101. The signal processing circuits may receive, amplify, filter and digitize signals from the catheter 101. The signals may be generated by sensors and/or a plurality of location electrodes located in the catheter 101.

Processing device 108 may also control other components of the system 100 according to the embodiments described herein. Processing device 108 is preferably programmed in software and/or hardware to perform the functions required by the system. The processing device 108 may store data for the software in a memory. The software may be downloaded to the processing device 108 in electronic form, over a network, or may be provided on tangible media, such as optical, magnetic or other nonvolatile memory media.

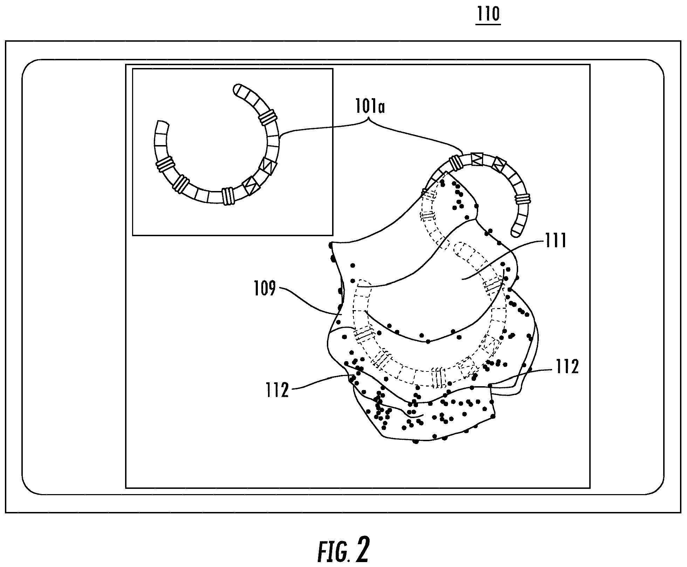

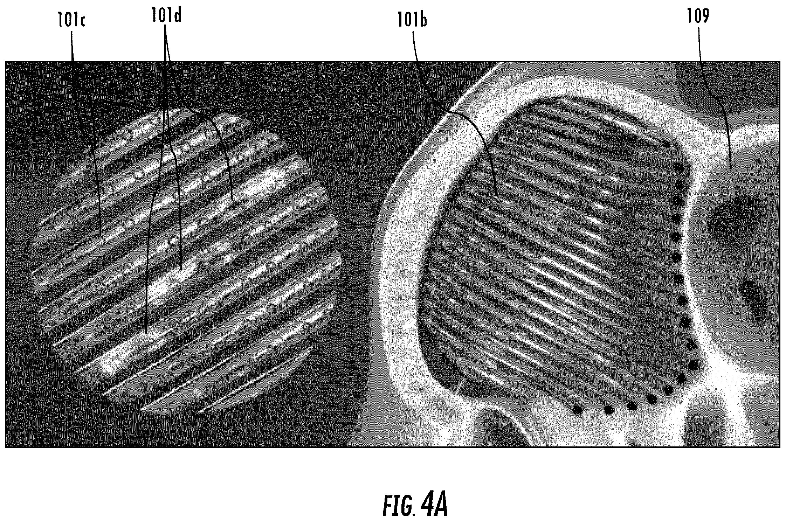

Catheter 101 is adapted for endocardial mapping and/or ablating cardiac tissue and includes a catheter body and a plurality of electrodes. The plurality of electrodes may be a plurality of mapping electrodes to measure electro-cardiac signals at one or more respective contact points with the heart tissue, and/or may be a plurality of ablation electrodes or electrodes capable of performing both mapping and ablation. Catheter 101 may be of any configuration that provides and/or enables coverage of the entire surface of the atrial chamber. Catheter 101 may be a lasso catheter comprising a number of non-overlapping loops, such as the lasso catheter shown in FIG. 2. Catheter 101 may be a basket catheter having a basket-shaped electrode array, such as the basket catheter shown in FIG. 4A. Basket catheters are known in the art and are described in U.S. patent No. 6,748,255, U.S. application publication No. 2016/0228023, and U.S. application publication No. 2006/0009690.

Catheter 101 may also comprise an array of temperature sensors for monitoring local tissue heating during ablation to prevent collateral damage to the epicardium, and adjacent tissue including the lungs or the esophagus. Catheter 101 may be inserted by an operator 104, who is typically a physician or a medical professional, into a chamber of the heart 111. Catheter 101 with a plurality of mapping electrodes may be used to prepare a functional electro-anatomic map of the heart of a patient as described, for example, in U.S. patent Nos. 6,892,091; 6,301,496 and 6,892,091. For example, the CARTO^{™} system, produced by Biosense Webster, Inc. (Diamond Bar, California) may be utilized for electro-anatomic mapping.

Processing device 108 may store mapping data in a memory. Processing device 108 may be coupled to a program operative to produce the visual display of the map 109 by driving a monitor 110. Map 109 may be an electro-anatomic map of the heart 111. Map 109 may be a 3-D map of the heart 111. Physician 104 may interact with the processing device 108 through graphical icons and visual indicators displayed on the monitor 110, for example, as described in U.S. application publication No. 2015/0057529. Physician 104 may view the electro-anatomic map 109 displayed on the screen of the processing device 108 and identify abnormal atrial tissue that may be targeted for ablation by application of thermal energy. The abnormal atrial tissue is also referred herein as target tissue. The target tissue may be tissue having abnormal electrical conductivity. The target tissue may be tissue initiating or triggering atrial fibrillation. The target tissue may be in the vicinity of the pulmonary veins.

Physician 104 may provide the system 100 with the parameters of the target tissue through visual indicators of the electro-anatomic map. The parameters of the target tissue may be, but are not limited to, tissue thickness and the presence of scar tissue. Physician 104 may draw a target line on a map 109 displayed on the monitor 110. As used herein, the target line may be a line drawn over target tissue displayed on the electro-anatomic map. Using the parameters provided by the physician 104, the processing device 108 may automatically adjust the time, energy level, and/or temperature of ablation.

Processing device 108 may also automatically select one or more electrodes of the plurality of the ablation electrodes that need to be activated based on the proximity to the target line. In addition, critical areas may be marked on the map 109 by the physician 104 and avoided by the system for safety. As used herein, "critical areas, or sites" refer to the areas of the heart which may be damaged as a result of an ablation procedure performed in the vicinity of the areas. Critical areas may be, for example, in proximity to the esophagus, vagal nerves, or phrenic nerves. For example, ablation procedures involving the posterior wall of the left atrium may cause esophageal damage and result in formation of an atrial esophageal fistula. If ablation must occur in the vicinity of the critical areas, the ablation time and energy used near these critical areas may be reduced, or ablation may be avoided. Alternatively, the processing device 108 may automatically determine the critical areas on the map 109 and may reduce the ablation energy and time limits during treatment. Catheter 101 may be used for mapping and for delivery of thermal energy during ablation. Catheter used for mapping (not shown) may be removed and replaced with an ablation catheter 101 which may be inserted in the same location of the heart using the same system of coordinates. Catheter 101 may be registered with CARTO^{™} system to perform the ablation.

Catheter 101 may also contain position sensors that provide signals to the processing device 108 in a console 102. For example, a magnetic field sensor, typically comprising coils, may be attached to the catheter 101 near a distal end 107. The position sensor may generate electrical position signals in response to the magnetic fields from the coils, thereby enabling processing device 108 to determine the coordinates, or position, of distal end 107 within the heart cavity 111, and thus the coordinates of each one of the plurality of the ablation electrodes. Catheter 101 may be configured to relay the coordinates of the multiple ablation sites formed by the plurality of the ablation electrodes to the processing device 108. Processing device 108 may be configured to receive signals from the magnetic field sensor signals indicative of the coordinates of the plurality of ablation electrodes of the catheter 101. The digitalized signals may be received and used by the console 102 and the positioning system to compute the position and orientation of the catheter 101, and analyze the electrical signals from the electrodes. Processing device 108 then may compute the position coordinates for each one of the plurality of the ablation electrodes. In other embodiments, the processing device 108 may be configured to receive the coordinates of the ablation sites recorded by any suitable imaging system. Processing device 108 may receive the coordinates of the multiple ablation sites by any suitable method in order to use the coordinates to identify the suitable electrodes to perform the ablation.

Position sensors may be included in a positioning sub-system. The positioning sub-system may be incorporated into the system 100. The positioning sub-system may be a magnetic-based or an impedance-based navigation system adapted for navigating the catheter. Console 102 may comprise a driver circuit, which drives magnetic field generators 106 placed at known positions external to a patient 103. The magnetic-based navigation system may determine the position and orientation of the catheter 101 by generating magnetic fields in a predefined working volume and sensing these fields at the catheter, using field generating coils. Alternatively, an impedance-based navigation system utilizing impedance measurement may be employed. Wire connections may link the console 102 with a plurality of body surface electrodes on a patient 103 typically attached to the patient's chest above the heart (not shown in FIG.1). The ablation electrodes and the body surface electrodes may be used to measure tissue impedance at an ablation site as described, for example, in U.S. patent Nos. 7,536,218 and 7,756,576. The positioning sub-system may be, for example, the CARTO^{™} system, produced by Biosense Webster Inc. (Diamond Bar, Calif.). For details see U.S. patent Nos. 5,391,199; 6,690,963; 6,484,118; 6,239,724; 6,618,612; 6,332,089, 6,690,963; 7,729,742; PCT application publication No. WO 1996/05768, and U.S. application publication No. 2004/0068178. The CARTO VISI TAG^{™} Module may provide a visual representation of ablation sites and may assist the physician 104 in designing successful ablation strategy and tailoring it to the anatomical location of the target tissue. The VISI TAG^{™} Module may assist the physician 104 in monitoring of power, contact force and time of ablation.

Processing device 108 may use the position of distal coordinates of the map points to construct a simulated surface of the portion of the heart 103. Processing device 108 then may combine the electrical potential measurements of the map points with the simulated surface to produce a map of the potentials overlaid on the simulated surface. System 100 may use an imaging technique to synchronize images of the heart with the mapping in the catheter position sensing system.

Console 102 may comprise one or more ablation power generators, which are used to apply energy to the plurality of the ablation electrodes of the catheter 101. Catheter 101 may be used to conduct any ablative energy to the target tissue in the heart. The power generator may selectively direct energy only to electrodes positioned in close proximity to the target tissues. The ablative energy may be radiofrequency energy, ultrasound energy, and laser-produced energy as described, for example, in U.S. patent Nos. 6,814,733; 6,997,924, and 7,156,816.

When the ablation electrode contacts the heart tissue, the ablative energy locally heats and induces a local necrosis of the heart tissue at the ablation site. The positioning sub-system records the position of the multiple ablation sites formed by the ablation electrodes during the procedure. The position of the ablation sites may also be observed by using an imaging system, such as ultrasound, fluoroscopy, or magnetic resonance imaging (MRI).

The system illustrated in FIG. 1 is provided merely for visual clarity and not by way of limitation of the embodiments described herein.

Physician 104 may acquire the image 109 of the heart and display the image 109 on the screen of the monitor 110. An exemplary screen display of the monitor 110 described herein is illustrated on FIG. 2. A functional electro-anatomic map 109 of the heart, in which local activation times are represented by different shading patterns, is shown. The screen display includes an image of lasso catheter 101a. Lasso catheter 101a within the heart 111 is partially obscured by the details of the electro-anatomic map 109 and target sites 112 previously obtained during mapping performed using mapping electrodes of the catheter 101a. The orientation of the catheter 101a and indications of the status of its electrodes are visible in the image of the catheter 101a. The physician 104 may mark the electro-anatomic map 109 with a target line, e.g., a line interconnecting the target sites 112.

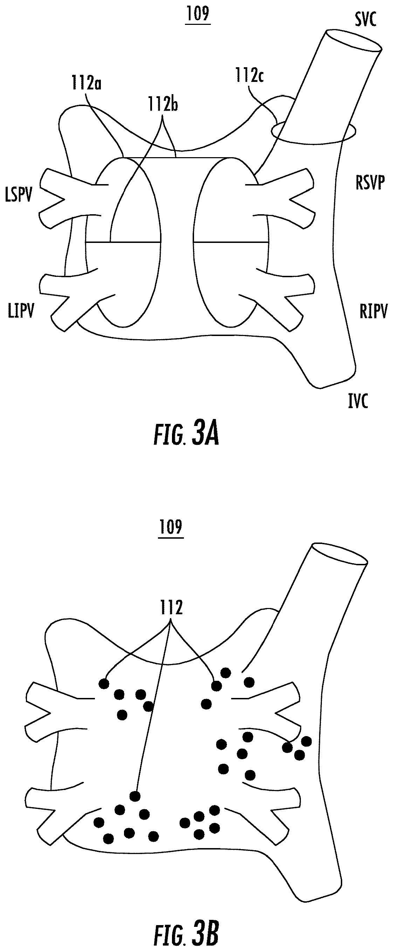

FIGS. 3A - 3B are views of the heart marked with target lines (FIG. 3A) and target sites (FIG. 3B) for automatic cardiac ablation. Referring to FIG. 3A, the electro-anatomic map 109 may be marked with the line 112a which encircles the left superior pulmonary vein (LSPV) and left inferior pulmonary vein (LIPV) or the right superior pulmonary vein (RSPV) and right inferior pulmonary vein (LIPV), or both left and right pulmonary veins. The electro-anatomic map may be marked with the line 112b which connects circles around left and right pulmonary veins, or creates a diameter within each of the circles around left and right pulmonary veins. The electro-anatomic map may be marked with the line 112c which encircles the superior vena cava (SVC). The electro-anatomic map may be marked with lines 112a, 112b, or 112c alone or in any combination. Referring to FIG. 3B, the processing device may automatically identify the target sites 112 on the electro-anatomic map.

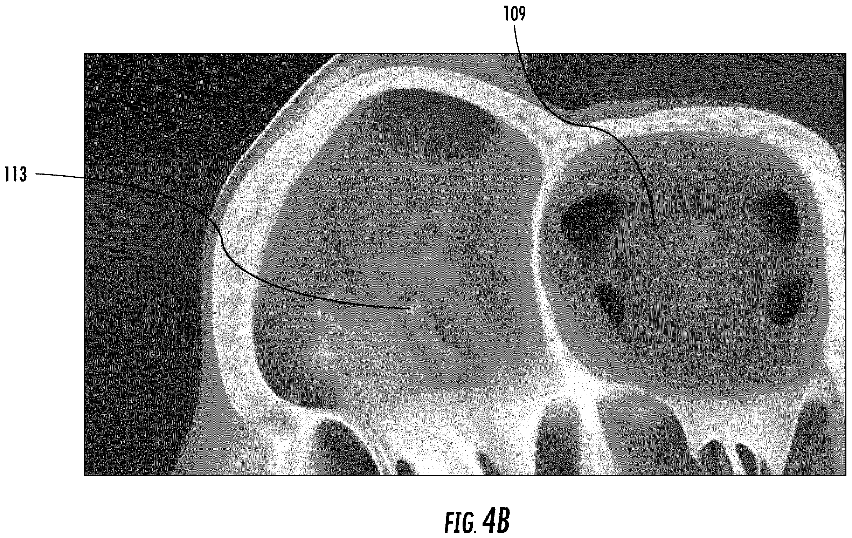

FIGS. 4A - 4B are views of the left atrium of the heart. FIG. 4A is a view of an exemplary basket catheter 101b for performing automatic cardiac ablation according to embodiments disclosed herein. The catheter body includes multiple splines, and each spline contains a plurality of ablation electrodes 101c. The catheter is configured to activate electrodes 112d in proximity to the target line of the heart 111 identified on the image of the heart 109 and perform automatic ablation. The catheter may include from 50 to 54 ablation electrodes. Those of skill in the art would realize that more or less electrodes may be included and that the positioning and spacing of the electrodes may vary. Each of the ablation electrodes may be 4 mm in size. The distance between two ablation electrodes positioned next to each other is from 3 to 4 mm. FIG. 4B is a view of an example ablation line 113 in the image of the heart 109 following the ablation procedure.

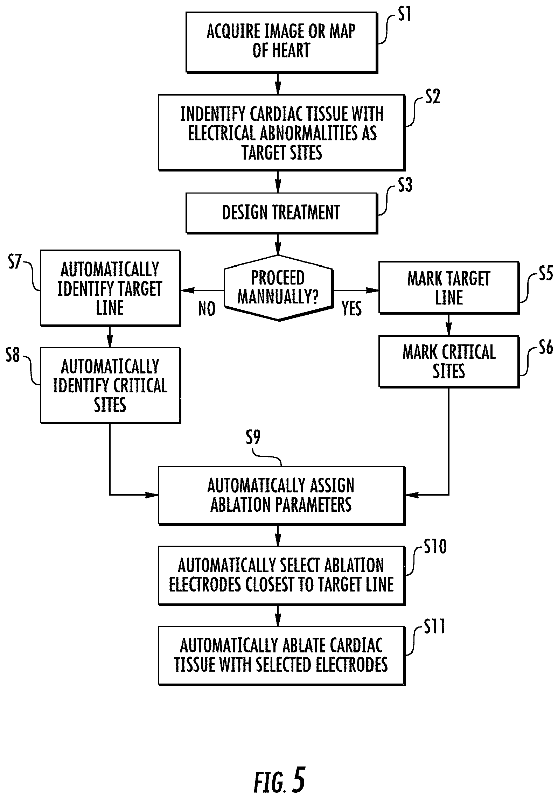

FIG. 5 is a flow chart diagram of the steps of an exemplary method for the map-guided automatic cardiac ablation, which method alone is not within the scope of the appended claims. For example, the method may comprise the step S1 of acquiring an image or map of a heart of a patient having a cardiac disorder and displaying the image on a visual display screen. The image may be a three-dimensional ultrasound image, MRI image or an image obtained by any other imaging technique known in the art. The image may be an electrical or electro-anatomic map 109 of the heart. The electro-anatomic map 109 may be acquired by using CARTO^{™} mapping and navigation system.

In step S2, the electro-anatomic map is evaluated to determine cardiac tissue exhibiting electrical abnormalities and determine target sites on the map that can be targeted for ablation.

In step S3, the treatment procedure can be designed. At this step, a catheter having a plurality of ablation electrodes can be registered with the positioning system and inserted into a portion of the heart having abnormal tissues.

In step S4, a decision whether to proceed manually and mark a target line for ablation, or whether to proceed automatically is made. If it is decided to proceed manually (S4=YES), in step S5, the ablation path is marked for ablating the identified target sites, for example, by drawing a target line interconnecting two or more target sites on the image of the heart 109. In an embodiment, the system may analyze the electro-anatomic map and recommend the position of the target line to physician 104. In another embodiment, physician 104 may identify target parameters for ablation of the target tissue. The target parameters may characterize cardiac tissue by, for example, indicating tissue thickness or the presence of scar tissue.

In step S6, physician 104 can mark one or more critical areas or sites on the image of the heart 109. Since the ablation process destroys the unwanted electrical pathways by heating local tissue to a temperature of irreversible damage, thereby forming non-conducting lesions, ablation at excessive temperature and duration may cause injury to adjacent tissue, including damage to the esophagus, lungs, phrenic nerve, or perforation of the heart wall. Accordingly, to avoid or minimize the damage, the critical sites indicated in step S6 can be ablated using "mild treatment parameters". The mild treatment parameters may be, for example, a reduced ablation time, lower energy, and/or lower temperature compared to the treatment parameters applied to a non-critical area chosen for ablation. Physician 104 can mark critical sites with another line where ablation may be performed using mild treatment parameters, or avoided.

If it is decided to proceed automatically (S4=NO), in step S7, a target line for ablation may be identified automatically. In step S8, critical sites may also be identified automatically.

Parameters for ablation may be assigned, based on the identified target lines. Note that processing device 108 can be integrated with the imaging system to facilitate processing of the information.

The image recognition algorithm performed by the processing device 108 may identify the target line marked by the physician 104 on the image 109 in step S5, or the processing device 108 may identify the target line automatically in step S7.

In step S9, the ablation parameters are calculated to facilitate automatic ablation of the corresponding tissue in the heart of a patient. The ablation parameters may be, but are not limited to, an ablation time, an ablation temperature, or an ablation energy. The ablation parameters may be adjusted in proximity to critical sites. The adjustment of ablation parameters may be reduction of time, temperature and/or energy of ablation, or entirely avoiding ablation.

In step S10, the processing device identifies ablation electrodes of the catheter that are closest to the target line. In an embodiment, ablation electrodes closest to the target line may be selected by physician 104.

In step S11, the selected electrodes are activated and ablation is performed according to the pre-determined parameters. The positioning system may be adapted to compare the target sites in the heart of a patient after ablation with the same sites before ablation.

Processing device 108 is used for all steps of the method described herein and typically comprises signal processing circuits with software and algorithms. Processing device 108 may be used to insert a catheter 101 into the heart 111 of the patient 103. Catheter 101 may have a plurality of ablation electrodes and a plurality of sensing electrodes. The positioning system, for example, such as CARTO^{™}, may be used to track the positions of the ablation electrodes and identify ablation electrodes in proximity of the target tissues.

It should be understood that many variations are possible based on the disclosure herein. Although features and elements are described above in particular combinations, each feature or element can be used alone without the other features and elements or in various combinations with or without other features and elements.

It should be understood that many variations are possible based on the disclosure herein. Although features and elements are described above in particular combinations, each feature or element can be used alone without the other features and elements or in various combinations with or without other features and elements.

The methods provided include implementation in a general purpose computer, a processor, or a processor core. Suitable processors include, by way of example, a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs) circuits, any other type of integrated circuit (IC), and/or a state machine. Such processors can be manufactured by configuring a manufacturing process using the results of processed hardware description language (HDL) instructions and other intermediary data including netlists (such instructions capable of being stored on a computer readable media). The results of such processing can be maskworks that are then used in a semiconductor manufacturing process to manufacture a processor which implements the methods described herein.

The methods or flow charts provided herein can be implemented in a computer program, software, or firmware incorporated in a non-transitory computer program, software, or firmware incorporated in a non-transitory computer-readable storage medium for execution by a general purpose computer or a processor. Examples of non-transitory computer-readable storage mediums include a ROM, a random access memory (RAM), a register, cache memory, semiconductor memory devices, magnetic media such as internal hard disks and removable disks, magneto-optical media, and optical media such as CD-ROM disks, and digital versatile disks (DVDs).

Certain terminology is used in the description herein for convenience only and is not limiting. The words "right," "left," "top," and "bottom" designate directions in the drawings to which reference is made. The words "a" and "one," as used in the claims and in the corresponding portions of the specification, are defined as including one or more of the referenced item unless specifically stated otherwise. This terminology includes the words above specifically mentioned, derivatives thereof, and words of similar import. The phrase "at least one" followed by a list of two or more items, such as "A, B, or C," means any individual one of A, B or C as well as any combination thereof.

Further embodiments herein may be formed by supplementing an embodiment with one or more element from any one or more other embodiment herein, and/or substituting one or more element from one embodiment with one or more element from one or more other embodiment herein.

It is understood, therefore, that this invention is not limited to the particular embodiments disclosed, but is intended to cover all modifications which are within the scope of the invention as defined by the appended claims.

## Claims

1. A system for automatically ablating target tissue in a heart (111) of a patient comprising:
a processing device (108) adapted to store programs in a memory, the programs configured to present an image (109) of a heart on a screen display (110), enable automatic or manual selection of the target tissue, designate locations of the target tissue on the image (109) of the heart, and identify ablation parameters for ablation of the target tissue;
a catheter (101) comprising a plurality of ablation electrodes (101c), wherein the plurality of the ablation electrodes (101c) is configured to be brought into contact with target tissue;
a navigator, wherein the navigator is configured to select automatically at least one ablation electrode of the plurality of the ablation electrodes (101c), the at least one ablation electrode being closest to the designated locations of the target tissue;
a generator adapted to automatically ablate the target sites in the heart; wherein the processing device (108) is adapted to drive the generator to ablate the target tissue with at least one selected ablation electrode.

2. The system of claim 1, wherein the image (109) comprises an electro-anatomic map of the heart.

3. The system of claim 1, wherein the catheter (101) is one of a lasso catheter or a basket catheter.

4. The system of claim 1, wherein the catheter (101) comprises at least thirty ablation electrodes.

5. The system of claim 1, wherein the one or more ablation parameters are selected from the group consisting of: ablation time, ablation temperature, and ablation energy.

6. The system of claim 1, wherein the navigator is a magnetic-based navigator or an impedance-based navigator.

7. The system of claim 1, wherein the processing device (108) is adapted to compare the target sites after ablation with the target sites before ablation and indicate differences.

8. A computer software product, including a non-transitory computer readable storage medium in which computer program instructions are stored, which instructions, when executed by a computer, cause the computer to perform the steps of:
acquiring an image of a heart of a subject having a cardiac disorder and displaying the image (109) on a screen display (110);
identifying a plurality of target sites (112) on the image (109), wherein each target site of the plurality of the target sites (112) is a cardiac tissue exhibiting electrical abnormality;
designing treatment comprising interconnecting two target sites of the plurality of target sites (112) by marking a target line on the image (109);
automatically selecting at least one ablation electrode of a plurality of ablation electrodes (101c) on a catheter in the heart of the subject, the at least one electrode being closest to the target line; and
automatically ablating the cardiac tissue by using the at least one selected ablation electrode.

## Patentansprüche

1. System zur automatischen Ablation von Zielgewebe in einem Herz (111) eines Patienten, das Folgendes umfasst:
eine Verarbeitungsvorrichtung (108), die dafür ausgelegt ist, Programme in einem Speicher zu speichern, wobei die Programme ausgebildet sind, um ein Bild (109) eines Herzes auf einer Bildschirmanzeige (110) darzustellen, eine automatische oder manuelle Auswahl des Zielgewebes zu ermöglichen, Positionen des Zielgewebes auf dem Bild (109) des Herzes zu bezeichnen und Ablationsparameter für die Ablation des Zielgewebes zu identifizieren;
einen Katheter (101), der eine Vielzahl von Ablationselektroden (101c) umfasst, wobei die Vielzahl der Ablationselektroden (101c) ausgebildet ist, um in Kontakt mit Zielgewebe gebracht zu werden;
einen Navigator, wobei der Navigator ausgebildet ist, um automatisch mindestens eine Ablationselektrode aus der Vielzahl der Ablationselektroden (101c) auszuwählen, wobei die mindestens eine Ablationselektrode den bezeichneten Stellen des Zielgewebes am nächsten ist;
einen Generator, der so ausgelegt ist, dass er die Zielstellen im Herz automatisch ablatiert; wobei die Verarbeitungsvorrichtung (108) so ausgelegt ist, dass sie den Generator antreibt, um das Zielgewebe mit mindestens einer ausgewählten Ablationselektrode zu ablatieren.

2. System nach Anspruch 1, wobei das Bild (109) eine elektro-anatomische Karte des Herzes umfasst.

3. System nach Anspruch 1, wobei der Katheter (101) entweder ein Lasso-Katheter oder ein Korb-Katheter ist.

4. System nach Anspruch 1, wobei der Katheter (101) mindestens dreißig Ablationselektroden umfasst.

5. System nach Anspruch 1, wobei der eine oder die mehreren Ablationsparameter ausgewählt sind aus der Gruppe bestehend aus: Ablationszeit, Ablationstemperatur und Ablationsenergie.

6. System nach Anspruch 1, wobei der Navigator ein magnetischer Navigator oder ein impedanzbasierter Navigator ist.

7. System nach Anspruch 1, wobei die Verarbeitungsvorrichtung (108) geeignet ist, die Zielstellen nach der Ablation mit den Zielstellen vor der Ablation zu vergleichen und Unterschiede anzuzeigen.

8. Computersoftwareprodukt, das ein nichtflüchtiges computerlesbares Speichermedium enthält, in dem Computerprogrammanweisungen gespeichert sind, wobei die Anweisungen, wenn sie von einem Computer ausgeführt werden, den Computer veranlassen, die folgenden Schritte durchzuführen:
Erfassen eines Bilds eines Herzes einer Testperson mit einer Herzerkrankung und Anzeigen des Bilds (109) auf einer Bildschirmanzeige (110);
Identifizieren einer Vielzahl von Zielstellen (112) auf dem Bild (109), wobei jede Zielstelle der Vielzahl der Zielstellen (112) ein Herzgewebe ist, das eine elektrische Abnormalität aufweist;
Entwerfen einer Behandlung, die das Verbinden von zwei Zielstellen der Vielzahl von Zielstellen (112) durch Markieren einer Ziellinie auf dem Bild (109) umfasst;
automatisches Auswählen mindestens einer Ablationselektrode aus einer Vielzahl von Ablationselektroden (101c) auf einem Katheter im Herz der Testperson, wobei die mindestens eine Elektrode der Ziellinie am nächsten ist; und
automatische Ablation des Herzgewebes unter Verwendung der mindestens einen ausgewählten Ablationselektrode.

## Revendications

1. Système d'ablation automatique d'un tissu cible dans le coeur (111) d'un patient comprenant :
un dispositif de traitement (108) adapté pour stocker des programmes dans une mémoire, les programmes étant configurés pour présenter une image (109) d'un coeur sur un écran (110), permettre une sélection automatique ou manuelle du tissu cible, désigner les emplacements du tissu cible sur l'image (109) du coeur, et identifier les paramètres d'ablation pour l'ablation du tissu cible ;
un cathéter (101) comprenant une pluralité d'électrodes d'ablation (101c), la pluralité d'électrodes d'ablation (101c) étant configurée pour être mise en contact avec le tissu cible ;
un navigateur, le navigateur étant configuré pour sélectionner automatiquement au moins une électrode d'ablation de la pluralité d'électrodes d'ablation (101c), l'au moins une électrode d'ablation étant la plus proche des emplacements désignés du tissu cible ;
un générateur adapté à l'ablation automatique des sites cibles dans le coeur ; le dispositif de traitement (108) étant adapté pour piloter le générateur afin de procéder à l'ablation du tissu cible à l'aide d'au moins une électrode d'ablation sélectionnée.

2. Système selon la revendication 1, dans lequel l'image (109) comprend une carte électro-anatomique du coeur.

3. Système selon la revendication 1, dans lequel le cathéter (101) est un cathéter lasso ou un cathéter à panier.

4. Système selon la revendication 1, dans lequel le cathéter (101) comprend au moins trente électrodes d'ablation.

5. Système selon la revendication 1, dans lequel les un ou plusieurs paramètres d'ablation sont sélectionnés dans le groupe constitué par : le temps d'ablation, la température d'ablation et l'énergie d'ablation.

6. Système selon la revendication 1, dans lequel le navigateur est un navigateur à base magnétique ou un navigateur à base d'impédance.

7. Système selon la revendication 1, dans lequel le dispositif de traitement (108) est adapté pour comparer les sites cibles après l'ablation avec les sites cibles avant l'ablation et indiquer les différences.

8. Produit logiciel informatique, comprenant un support de stockage lisible par ordinateur, non transitoire, sur lequel sont stockées des instructions de programme informatique, lesquelles instructions, lorsqu'elles sont exécutées par un ordinateur, amènent l'ordinateur à exécuter les étapes suivantes :
acquérir une image du coeur d'un sujet souffrant d'un trouble cardiaque et afficher l'image (109) sur un écran (110) ;
identifier une pluralité de sites cibles (112) sur l'image (109), chaque site cible de la pluralité de sites cibles (112) étant un tissu cardiaque présentant une anomalie électrique ;
concevoir un traitement comprenant l'interconnexion de deux sites cibles de la pluralité de sites cibles (112) en marquant une ligne cible sur l'image (109) ;
sélectionner automatiquement au moins une électrode d'ablation parmi une pluralité d'électrodes d'ablation (101c) sur un cathéter dans le coeur du sujet, l'au moins une électrode étant la plus proche de la ligne cible ; et
procéder à l'ablation automatique du tissu cardiaque à l'aide de l'au moins une électrode d'ablation sélectionnée.
